# EUROPEAN PATENT SPECIFICATION

(11) **EP 0 540 507 B1**
(45) Date of publication and mention of the grant of the patent: **29.11.1995**
(21) Application number: 93200021.9
(22) Date of filing: 16.03.1990
(51) Int. Cl.: A61M 39/04

(54) **Blunt-ended cannula device**
Stumpfer Einstechdorn
Aiguille émoussée

(30) Priority: 17.03.1989 US 325617
(43) Date of publication of application: 05.05.1993
(62) Divisional of application: 90905119.5
(73) Proprietor: BAXTER INTERNATIONAL INC., Deerfield, Illinois 60015 (US)
(72) Inventor: Jepson, Steven C., Palatine, Illinois 60067 (US); Dudar, Thomas E., Palatine, Illinois 60067 (US); Finley, Michael J., Park City, Illinois 60085 (US); Desecki, Vincent C., Ingleside, Illinois 60041 (US)
(74) Representative: MacGregor, Gordon

(56) References cited:
- EP-A- 0 220 911
- WO-A-86/01487
- DE-U- 8 425 197
- US-A- 4 804 366
- US-A- 4 834 716

## Description

The present invention relates to a blunt-ended cannula device for use with a pre-slit resealable injection site.

An injection site usable with a blunt-ended cannula is known from US-A-4197848. A blunt-ended cannula can be forced through the sealing member placing the cannula in fluid flow communication with a fluid flow pathway in the injection site. A prior art blunt-ended cannula is illustrated in Fig 1 and described hereafter. EP-A-0220911 discloses a blunt-ended cannula device including a cylindrical sheath surrounding the cannula. The precharacterising part of Claim 1 is based on this disclosure.

The distinguishing features of the invention are as set out in the characterising part of Claim 1.

The present invention is directed to a cannula device which is usable with an in-line injection site and with a syringe and which has its cannula protected from inadvertent touch contamination.

Reference is now made to the accompanying drawings, wherein:-
Fig 1 is a side elevational view, partly in section, of a prior art preslit injection site and associated blunt-ended cannula;
Fig 2 is a perspective view, partially broken away, showing a combination of a syringe with a blunt-ended cannula device according to the invention;
Fig 3 is a cross-sectional view of a pre-slit, in-line injection site shown with an inserted blunt-ended cannula, the injection site being usable with the cannula device of Fig 2;
Fig 4 is a perspective view of a tip protector for attachment over the end of the blunt cannula device of Fig 2;
Fig 5 is a cross-sectional view of a blunt-ended cannula device, which is not in accordance with the present invention, but includes a feature usable in the present invention; and
Fig 6 is a perspective view of the cannula device of Fig 2 in joined relationship with the injection site of Fig 3.

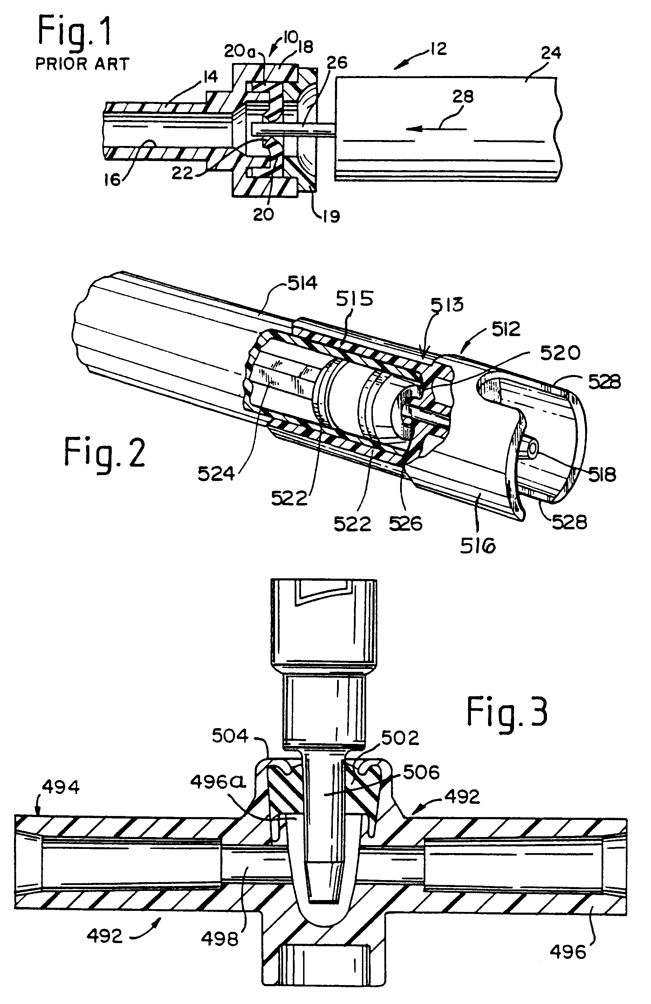

A prior art pre-slit injection site 10 and associated blunt cannula 12 are illustrated in Figure 1. The prior art injection site 10 has a cylindrical housing 14 with a fluid flow path 16 therethrough. A first end 18 of the housing 14 is closed with a relatively thin disc-shaped resealable member 20. The member 20 has a resealable opening 22 therein.

The member 20 is a molded septum with an integrally formed skirt 20a. The skirt 20a is oriented generally perpendicular to the portion of the septum with the opening 22.

The cannula 12 includes a body portion 24 which carries at a first end a hollow, cylindrical, blunt piercing member 26. As the cannula 12 is moved in a direction 28 toward the first end 18 of the injection site 10, the member 26 slidably engages the opening 22. The sealing member 20 is then deformed adjacent the opening 22 and the number 26 extends into the flow path 16. A fluid flow path through the cannula 12 will then be in fluid flow communication with the flow path 16 via the hollow piercing member 26.

Reference is made to WO-A-8906553 (EP-0354947) published 21 February 1990. This document teaches various constructions of pre-slit injection sites and blunt cannula devices as well as methods of mounting a septum. The disclosure of this document provides a full enabling disclosure for making pre-slit injection sites and blunt cannula devices and is incorporated in the present specification by reference, to avoid repetition of the matter disclosed therein.

Fig 3 shows, an in-line pre-slit injection site 492, preferably for adding medication to a fluid stream, removing a sample from a fluid stream, or similar application. The site depicted in Figure 3 has a fluid entry port 494 at one end, a fluid exit port 496 at the other end, and a fluid passageway 498 communicating directly between the entry and exit ports. The inlet and outlet may have such additional features as are useful for connecting the injection site device within a fluid flow path. As depicted, the inlet defines a slightly tapered female surface and the outlet defines a similarly female tapered surface which are preferably joined by solvent bonding a similar attachment to plastic tubing of an administration set, extension set or the like. Standard luer fittings or surfaces could also be provided at the inlet or outlet, as desired.

For injecting liquid into the fluid stream or sampling the fluid stream, the device has a side channel 496a which communicates between a pre-slit septum 502 and the fluid passageway 498. The septum 502 is made as described in EP-A-0354947 and mounted and held in position by a swaged-over wall 504, which may include a colored identifier ring around the septum.

A blunt cannula, such as cannula 506, may be inserted through the pre-slit septum for injecting fluid into the liquid stream flowing between the inlet and outlet, or for taking samples of the fluid stream.

Figure 2 depicts a blunt-ended cannula device according to the present invention. The device is usable with the in-line injection site of Fig 3. Fig 2 depicts a blunt cannula device 512 in combination with a syringe 514. The blunt cannula device 512 has a generally cylindrical outer wall 513 defining a sheath 516 which encloses and substantially protects a blunt cannula portion 518. The blunt cannula portion is attached to and extends from an intermediate transverse interior wall 520. The blunt cannula device 512 may be attached to a syringe in various ways. As depicted, however, the syringe 514 has a glass barrel wall which is tightly press fit into a portion 515 of the cylindrical outer wall, extending in the opposite direction to the sheath 516, the syringe extending in the cylindrical wall portion 515 up to the transverse wall 520.

Although various syringes may be used in connection with the blunt cannula device 512, the syringe depicted in Figure 2 is of the type prefilled with a medical liquid such as heparin. The syringe depicted in Figure 2 has a pair of resilient pistons 522 spaced apart, with the fluid to be dispensed contained between the pistons. A plunger rod 524 pushes the pistons forward until the forward most piston engages against an entry port 526 which extends in a direction opposite the blunt cannula 518. The forwardmost piston has a frangible portion, which is pierced by the entry port, releasing the liquid contained between the pistons for expulsion through the blunt cannula.

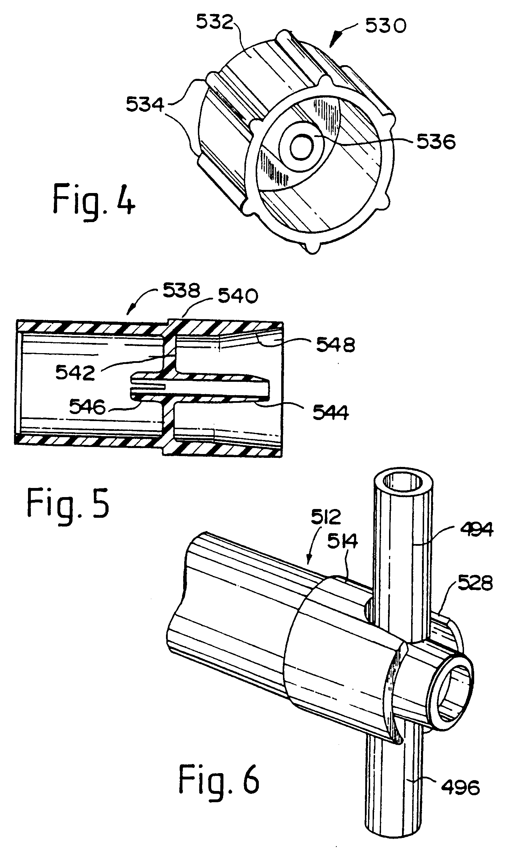

The blunt cannula portion 518 is substantially protected from inadvertent touch contamination by the shield 516 of the outer cylindrical wall 513. To permit the blunt cannula to be used, however, with the in-line injection site 492 or a similar device, a pair of opposed, generally U-shaped recesses 528 are provided in the shield for receiving the inlet and outlet portions 494, 496 of the in-line injection site when the cannula is attached to it. This arrangement is depicted in a perspective view in Figure 6. As shown there, the blunt cannula device 512 may be attached to the in-line injection site by inserting the blunt cannula portion into the pre-slit injection site, with the U-shaped recesses 528 receive the inlet and outlet portions 494, 496 of the in-line injection site, thus allowing the bare cannula to be inserted sufficiently far into the pre-slit injection site.

Figure 4 shows a tip protector 530 for the blunt cannula device. The tip protector 530 has a generally cylindrical outer wall 532 with raised ribs 534 for gripping. The cylindrical wall is sized to slip over the end cylindrical wall 514 of the blunt cannula device 512, and is sufficiently long to extend beyond the U-shaped recesses to completely enclose and protect the blunt cannula 518 during shipping, storing and between uses, if so desired.

The protector does not form part of the present invention.

Concentrically disposed within the cylindrical wall 532, the tip protector has an axially extending, hollow tube 536 for slidably receiving the blunt cannula 518 therewithin. The shield or tip protector 530 would typically be attached to the blunt cannula device 512 during manufacture, and removed when the syringe and blunt cannula device are used. If so desired, it may be re-attached between uses to protect the cannula from any further contamination.

Figure 5 shows a blunt cannula device, which is not in accordance with the present invention. As shown in Figure 5, the blunt cannula device 538 has a cylindrical outer wall 540, a transverse intermediate inner wall 542, a blunt cannula 544 extending axially from the transverse intermediate wall and an entry port 546 extending in the opposite direction from the blunt cannula. The difference between this device and the one shown in Figure 2 is the absence of U-shaped recesses for use with an in-line injection site such as depicted in Figure 6. For ease of attachment to an injection site, the inner surface of the cylindrical wall is tapered at 548.

## Claims

1. A blunt-ended cannula device for use with a pre-slit resealable injection site (492), the cannula device (512) comprising an elongated member (518) with a fluid flow channel extending generally axially therewithin and through a distal end of the member, the member being generally cylindrical along a substantial portion of its length and terminating in a generally tapered distal end portion having a blunt end edge, a substantially cylindrical sheath (516) which surrounds the elongate member (518) and is at least co-extensive with and spaced apart from the elongated member to protect the member from inadvertent touch contamination, characterised in that the distal end of the sheath has at least two slots (528) opposite each other, and
the interior surface (548) of the sheath around the elongate member is outwardly tapered.

2. A blunt-ended cannula device according to Claim 1, further comprising a generally cylindrical wall portion (515) extending in the opposite direction to the sheath to receive the end of a syringe (514).

3. A blunt-ended cannula device according to Claim 2, in combination with a syringe (514) having a barrel wall press-fitted into said cylindrical wall portion (515).

4. A blunt-ended cannula device according to Claims 1, 2, or 3, in combination with an in-line injection site (492) having inlet and outlet portions (494,496) received in respective slots (528) of the sheath (516).

## Patentansprüche

1. Kanüleneinrichtung mit stumpfem Ende zur Verwendung mit einer vorgeschlitzten wiederabdichtbaren Injektionsstelle (492), wobei die Kanüleneinrichtung (512) folgendes aufweist:
- ein langgestrecktes Teil (518) mit einem Fluidströmungskanal, der sich im allgemeinen axial darin und durch ein distales Ende des Teiles hindurch erstreckt, wobei das Teil entlang eines wesentlichen Bereiches seiner Länge im allgemeinen zylindrisch ist und in einem im allgemeinen konisch zulaufenden distalen Endbereich endet, der eine stumpfe Endkante aufweist, und
- eine im wesentlichen zylindrische Hülse (516), die das längliche Teil (518) umgibt und die sich zumindest gemeinsam mit diesem erstreckt und von dem langgestreckten Teil beabstandet ist, um das Teil gegen Verunreinigungen durch unachtsame Berührung zu schützen,
dadurch gekennzeichnet,
daß das distale Ende der Hülse wenigstens zwei Schlitze (528) aufweist, die einander gegenüberliegen, und daß die Innenoberfläche (548) der Hülse um das langgestreckte Teil herum nach außen hin konisch zuläuft.

2. Kanüleneinrichtung mit stumpfem Ende nach Anspruch 1, die ferner einen im allgemeinen zylindrischen Wandbereich (515) aufweist, der sich in entgegengesetzter Richtung zu der Hülse erstreckt, um das Ende einer Spritze (514) aufzunehmen.

3. Kanüleneinrichtung mit stumpfem Ende nach Anspruch 2, in Kombination mit einer Spritze (514), die eine trommelförmige Wand aufweist, welche mit einer Preßpassung in den zylindrischen Wandbereich (515) eingesetzt ist.

4. Kanüleneinrichtung mit stumpfem Ende nach den Ansprüchen 1, 2 oder 3,
in Kombination mit einer in der Leitung liegenden Injektionsstelle (492), die Einlaß- und Auslaßbereiche (494, 496) aufweist, die in den jeweiligen Schlitzen (528) der Hülse (516) aufgenommen sind.

## Revendications

1. Dispositif à aiguille émoussée utilisable avec un site d'injection préfendu refermable (492), le dispositif à aiguille (512) comprenant un élément allongé (518) qui comporte un passage d'écoulement de fluide s'étendant sensiblement axialement dans ledit élément et à travers une extrémité distale de celui-ci, l'élément étant sensiblement cylindrique sur une partie importante de sa longueur et se terminant par une partie d'extrémité distale sensiblement conique et ayant un bord d'extrémité émoussé , et une gaine sensiblement cylindrique (516) qui entoure l'élément allongé (518) et est au moins de même longueur que l'élément allongé et à une certaine distance de celui-ci de manière à protéger ledit élément contre une contamination par contact accidentel, caractérisé en ce que l'extrémité distale de la gaine comporte au moins deux évidements (528) mutuellement opposés et en ce que la surface intérieure (548) de la gaine, autour de l'élément allongé, est évasée vers l'extérieur.

2. Dispositif à aiguille émoussée suivant la revendication 1, comprenant en outre une paroi sensiblement cylindrique (515) qui s'étend dans la direction opposée à celle de la gaine, pour recevoir l'extrémité d'une seringue (514).

3. Dispositif à aiguille émoussée suivant la revendication 2, en combinaison avec une seringue (514) ayant une paroi de tube emmanchée à la presse à l'intérieur de ladite paroi cylindrique (515).

4. Dispositif à aiguille émoussée suivant les revendications 1,2 ou 3, en combinaison avec un site d'injection en ligne (492) qui comprend des parties d'entrée et de sortie (494,496) reçues dans des évidements respectifs (528) de la gaine (516).
